# EUROPÄISCHE PATENTSCHRIFT

(11) **EP 2 550 980 B1**
(45) Veröffentlichungstag und Bekanntmachung des Hinweises auf die Patenterteilung: **20.09.2017**
(21) Anmeldenummer: 11009996.7
(22) Anmeldetag: 20.12.2011
(51) Int. Cl.: A61L 24/06, A61L 27/16, A61L 24/00, A61L 27/50

(54) **Pastenförmiger Knochenzement**
Bone cement in paste form
Ciment osseux pâteux

(30) Priorität: 27.07.2011 DE 102011108574
(43) Veröffentlichungstag der Anmeldung: 30.01.2013
(73) Patentinhaber: Heraeus Medical GmbH, 61273 Wehrheim (DE)
(72) Erfinder: Vogt, Sebastian, Dr., 99092 Erfurt (DE)
(74) Vertreter: Heraeus IP

(56) Entgegenhaltungen:
- WO-A2-2010/000384
- DE-A1-102007 050 763
- US-A- 5 166 117

## Beschreibung

Die vorliegende Erfindung betrifft einen Kit, sowie die Verwendung eines Kits zur Herstellung einer Paste zur mechanischen Fixierung von Gelenkprothesen, zur Abdeckung von Schädeldefekten, zur Auffüllung von Knochenkavitäten, zur Femuroplastie, zur Vertebroplastie, zur Kyphoplastie, zur Herstellung von Spacern oder zur Herstellung von Trägermaterialien für die lokale Antibiotika-Therapie.

Konventionelle Polymethylmethacrylat-Knochenzemente (PMMA-Knochenzemente) sind seit Jahrzehnten bekannt und gehen auf die grundlegenden Arbeiten von Sir Charnley zurück (Charnley, J.: "Anchorage of the femoral head prosthesis of the shaft of the femur"; J. Bone Joint Surg. 42 (1960) 28-30). Der Grundaufbau der PMMA-Knochenzemente ist seitdem prinzipiell gleich geblieben. PMMA-Knochenzemente bestehen aus einer flüssigen Monomerkomponente und einer Pulverkomponente. Die Monomerkomponente enthält im Allgemeinen (i) das Monomer Methylmethacrylat und (ii) einen darin gelösten Aktivator (zum Beispiel N,N-Dimethyl-p-toluidin). Die Pulverkomponente umfasst (i) ein oder mehrere Polymere, die auf Basis von Methylmethacrylat und Comonomeren, wie Styrol, Methylacrylat oder ähnlichen Monomeren, durch Polymerisation, vorzugsweise durch Suspensionspolymerisation, hergestellt sind, (ii) einen Röntgenopaker und (iii) einen Initiator (zum Beispiel) Dibenzoylperoxid. Beim Vermischen der Pulverkomponente mit der Monomerkomponente entsteht durch Quellung der Polymere der Pulverkomponente im Methylmethacrylat der Monomerkomponente ein plastisch verformbarer Teig. Gleichzeitig reagiert der Aktivator N,N-Dimethyl-p-toluidin mit Dibenzoylper- oxid, das unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs bis der Zementteig erstarrt und damit ausgehärtet ist.

Der wesentliche Nachteil der bisherigen PMMA-Knochenzemente für den medizinischen Anwender besteht darin, dass der Anwender die flüssige Monomerkomponente mit der Pulverkomponente unmittelbar vor der Zementapplikation in einem Mischsystem oder in Tiegeln vermischen muss. Dabei können leicht Mischungsfehler auftreten, welche die Zementqualität negativ beeinflussen. Ferner muss das Vermischen der Komponenten zügig erfolgen. Dabei kommt es darauf an, dass das gesamte Zementpulver ohne Klumpenbildung mit der Monomerkomponente vermischt wird und das beim Vermischungsvorgang der Eintrag von Luftblasen vermieden wird. Bei der Verwendung von Vakuummischsystemen wird im Gegensatz zur Handanmischung die Bildung von Luftblasen im Zementteig weitgehend verhindert. Beispiele für Mischsysteme sind in den Schriften US 4,015,945, EP-A-0 674 888 und JP 2003-181270 offenbart. Vakuummischsysteme machen jedoch eine zusätzliche Vakuumpumpe erforderlich und sind daher relativ kostenintensiv. Nach der Vermischung der Monomerkomponente mit der Pulverkomponente muss ferner je nach Zementtyp eine gewisse Zeit gewartet werden, bis der Zementteig klebfrei ist und appliziert werden kann. Aufgrund der vielen Fehlermöglichkeiten beim Anmischen von konventionellen PMMA-Knochenzementen wird zudem entsprechend geschultes Personal benötigt. Die entsprechenden Schulungen sind mit einem erheblichen Kostenaufwand verbunden. Weiterhin kommt es bei der Vermischung der flüssigen Monomerkomponente mit der Pulverkomponente zu einer Belastung der Anwender durch Monomerdämpfe und durch Freisetzung von pulverförmigen Zementpartikeln.

Als Alternative zu den konventionellen Pulver-Flüssigkeits-Polymethylmethacrylatknochenzementen wurden pastenförmige Polymethylmethacrylatknochenzemente in den Offenlegungsschriften DE-A-10 2007 052 116, DE-A-10 2007 050 762 und DE-A-10 2007 050 763 beschrieben. Dabei werden die Knochenzemente als vorgemischte, lagerstabile Pasten dem Anwender zur Verfügung gestellt. Diese Pasten enthalten jeweils ein radikalisch polymerisierbares Methacrylatmonomer, ein in diesem Methacrylatmonomer lösliches Polymer und ein in diesem Methacrylatmonomer unlösliches partikuläres Polymer (da beide Pasten ein unlösliches partikuläres Polymer beinhalten, werden solche Systeme als "symmetrisch" bezeichnet). In einer dieser Pasten ist zudem ein radikalischer Polymerisationsinitiator enthalten, während die andere Paste einen Polymerisationsaktivator aufweist. Der aus diesen Pasten hergestellte Knochenzement weist als Folge der gewählten Zusammensetzung eine ausreichend hohe Viskosität und Kohäsion auf, um dem Blutungsdruck bis zur Aushärtung standzuhalten. Beim Vermischen der beiden Pasten reagiert der Polymerisationsinitiator mit dem Akzelerator unter Bildung von Radikalen, die die radikalische Polymerisation der Methacrylatmonomere initiieren. Durch die fortschreitende Polymerisation härtet die Paste unter Verbrauch der Methacrylatmonomere aus. Es wurde gefunden, dass auch dann, wenn als im Methacrylatmonomer unlösliches partikuläres Polymer hochvernetzte Poly(methylmethacrylat)-Partikel verwendet werden, diese geringe Anteile an Methacrylatmonomer und darin gelösten Verbindungen aufnehmen und einschließen. Dadurch enthalten die unlöslichen Polymerpartikel der einen Paste Einschlüsse aus Monomerflüssigkeit und darin gelöstem Initiator, während die unlöslichen Polymerpartikel der anderen Paste ihrerseits Einschlüsse aus Monomerflüssigkeit und darin gelöstem Akzelerator enthalten. Nach dem Vermischen der beiden Pasten härtet die aus dem Methacrylatmonomer und dem gelösten Polymer bestehende Phase, in der die unlöslichen Polymerpartikel suspendiert sind, unter Herstellung von applikationsfähigem Knochenzement aus. In der Folgezeit diffundiert die zunächst eingeschlossene Monomerflüssigkeit aus den unlöslichen Polymerpartikeln heraus und polymerisiert nach. Die aus den unlöslichen Polymerpartikeln herausdiffundierende Monomerflüssigkeit wirkt bis zu ihrem Verbrauch infolge der Nachpolymerisation als Weichmacher. Dies führt dazu, dass die initial ausgehärteten Knochenzementpasten zwar die Anforderungen der ISO5833 erfüllen, jedoch noch eine ausgeprägte Nachhärtung infolge der Nachpo- lymerisation der aus den unlöslichen Polymerpartikeln diffundierenden Monomerflüssigkeit zeigen.

Das bei den bisherigen aus einer Pulverkomponente und einer Monomerflüssigkeit bestehenden PMMA-Knochenzementen eingesetzte Initiatorsystem Dibenzoylperoxid und N,N-Dimethyl-p-toluidin hat sich prinzipiell bewährt (K.-D. Kühn: Knochenzemente für die Endoprothetik: ein aktueller Vergleich der physikalischen und chemischen Eigenschaften handelsüblicher PMMA-Zemente. Springer-Verlag Berlin Heidelberg New York, 2001). Das Dibenzoylperoxid liegt dabei als Feststoff im Zementpulver vor und das N,N-Dimethyl-p-toluidin ist in der Monomerkomponente gelöst. Eigene Versuche mit Zementpasten unter Verwendung des Initiatorsystems Dibenzoylperoxid/N,N-Dimethyl-p-toluidin zeigten allerdings, dass Pasten mit N,N-Dimethyl-p-toluidin eine ausgeprägte Tendenz zur Spontanpolymerisation besitzen. Weiterhin wird der bei konventionellen Pulver/Flüssigkeits-Polymethylmethacrylat-Knochenzementen bewährte Beschleuniger N,N-Dimethyl-p-toluidin toxikologisch kritisch diskutiert.

Neben diesem Redoxsystem wurden auch Initiatorsysteme beschrieben, die auf der Verwendung von Barbituraten beruhen. In der DE-A-1 495 520 wurde ein Verfahren zur Polymerisation von Vinylverbindungen und Polyestern beschrieben. Dabei werden Barbitursäurederivate, Halogenid-Ionendonatoren und Kupferverbindungen im Monomer oder Monomergemisch gelöst. Die Kombination Barbitursäurederivat, Halogenid-Ionendonator und Kupferverbindung initiiert dabei die Polymerisation. Es können dazu auch organische Peroxide oder Wasserstoffperoxid zugesetzt werden. Eigene Versuche dazu zeigten, dass, entgegen der Annahme in der DE-A-1 495 520, wonach Luft oder Peroxide zur Auslösung der Polymerisation durch Barbiturat in Gegenwart von Kupfer-Ionen und ChloridIonen notwendig sind, auch ohne Luftsauerstoff oder Peroxide eine Initiierung möglich ist. Das bedeutet, dass offensichtlich das Barbiturat selbst als Initiator wirkt.

Ein etwas komplexeres System wird in der WO2010/000384 A2, der DE -A-10 2007 050 763 und der DE -A-10 2007 050 763 beschrieben. Bei diesem System sind Erdalkalisalze von Barbituraten und basische Kupfersalze in einer Paste enthalten. Diese beiden Salze sind im Methacrylat-Monomeren unlöslich. In einer zweiten Paste ist eine schwache organische Säure, wie 2-Ethylhexansäure, enthalten. Daneben ist noch ein Chlorid-Ionendonator in den Pasten vorhanden, vorzugsweise Tetraalkylammoniumchlorid als Chlorid-Ionendonator eingesetzt wird. Bei Vermischung der beiden Pasten wird durch die schwache organische Säure synchron sowohl das Barbiturat in die lösliche Säureform als auch das Kupfer in ein lösliches Kupfersalz überführt. Der Vorteil dieses Systems ist insbesondere bei Pasten mit mehrfachfunktionellen Monomeren, dass durch vorgelagerte Diffusions- und Ionenaustauschprozesse eine Erhöhung der Verarbeitungszeit möglich ist, die sonst bei der Verwendung von mehrfach funktionellen Monomeren nur sehr kurz ist und üblicherweise im Sekundenbereich liegt. Nachteilig an dem in der DE -A-10 2007 050 762 und der DE -A-10 2007 050 763 beschriebenen Initiatorsystem ist allerdings der Einsatz von Tetraalkylammoniumchlorid als Chlorid-IonenDonator, da es in Gegenwart von gelösten Schwermetallsalzen eine Spontanpolymerisation auslösen kann.

Der vorliegenden Erfindung lag die Aufgabe zugrunde, die sich aus dem Stand der Technik ergebenden Nachteile im Zusammenhang mit auf mindestens zwei Pasten basierenden Knochenzement-Systemen zu überwinden.

Insbesondere lag der vorliegenden Erfindung die Aufgabe zugrunde, einen Kit auf der Basis zweier Pasten bereitzustellen, der zur Herstellung von Knochenzement mit einer hohen initialen Festigkeit und damit einer geringen Nachhärtung vorgesehen ist. Auch sollten sich die Pasten, solange sie getrennt voneinander vorliegen, durch eine möglichst hohe Polymerisationsstabilität auszeichnen (also möglichst nicht zur Spontanpolymerisation neigen). 1 Kit zur Herstellung eines Knochenzementes, aufweisend eine Paste A und eine Paste B, wobei
(a) Paste A
(a1) wenigstens ein Methacrylat-Monomer, und
(a2) als Polymerisationsinitiator wenigstens ein Barbitursäurederivat beinhaltet;
(b) Paste B
(b1) wenigstens ein Methacrylat-Monomer, und
(b2) als Polymerisationsbeschleuniger wenigstens eine basische Schwermetallverbindung, die aus der Gruppe bestehend aus Schwermetallsalzen und Schwermetallkomplexen ausgewählt ist, beinhaltet;
und wobei
i) Paste B weniger als 0,01 Gew.-%, bezogen auf das Gesamtgewicht der Paste B, eines Peroxids beinhaltet,
ii) mindestens eine der Pasten A und B als Komponente (a3) bzw. (b3) wenigstens einen in (al) bzw. (b1) schwer- oder unlöslichen Füllstoff beinhaltet,
iii) mindestens eine der Pasten A und B als Komponente (a4) bzw. (b4) wenigstens ein anorganisches Halogenid-Salz beinhaltet, wobeidas wenigstens eine anorganische Halogenid-Salz (a4) bzw. (b4) ausgewählt ist aus der Gruppe bestehend aus einem Alkalihalogenid, einem Erdalkalihalogenid, und einer Mischung aus mindestens zwei dieser anorganischen Halogenid-Salze, und
iv) wobei Paste B weniger als 2 Gew.-% einer organischen Säure enthält, die in Monomer (b1) löslich ist.

Die Erfindung basiert unter anderem auf der Idee, eine erste Paste zu verwenden, die ein in einem radikalisch polymerisierbaren Monomer, wie Methylmethacrylat, lösliches Barbiturat enthält. Bei Vermischung dieser ersten Paste mit einer zweiten Paste, welche neben dem radikalisch polymerisierbaren Monomer eine Schwermetallverbindung enthält, reagiert das lösliche Barbiturat auf Grund seiner Acidität mit dem vorzugsweise basischen Schwermetallsalz. Überraschend zeigte sich, dass dadurch die Polymerisations- reaktion in Gegenwart eines anorganischen, vorzugsweise in dem Monomer löslichen Halogenidionen-Donators initiiert werden kann. Durch Einwirkung des Barbiturats auf das vorzugsweise basische Schwermetallsalz werden die Schwermetallionen offensichtlich in eine lösliche Salzform überführt, welche durch Einwirkung auf die Barbitursäure die Polymerisation des Methacrylatmonomeren initiiert. Die Verwendung von aromatischen Aminen und quartären Ammoniumchloriden als Beschleuniger ist dabei nicht mehr notwendig.

Erfindungsgemäß wird unter Kit ein System aus wenigstens zwei Komponenten verstanden. Obwohl im Folgenden von zwei Komponenten (i. e. Paste A und Paste B) die Rede ist, kann der Kit falls notwendig auch mehr als zwei Komponenten, beispielsweise drei, vier, fünf oder mehr als fünf Komponenten enthalten. Die einzelnen Kitkomponenten liegen vorzugsweise getrennt voneinander verpackt vor, so dass die Inhaltsstoffe der einen Kitkomponente mit den Inhaltsstoffen einer weiteren Kitkomponente nicht in Kontakt stehen. Beispielsweise ist es möglich, die jeweiligen Kitkomponenten getrennt voneinander zu verpacken und zusammen in einem Vorratsbehälter aufzubewahren.

Paste A beinhaltet als Komponente (a1) ein radikalisch polymerisierbares Monomer, wobei es sich vorzugsweise um ein Monomer handelt, welches bei einer Temperatur von 25°C und einem Druck von 1.013 hPa flüssig ist.

Das radikalisch polymerisierbare Monomer (a1) ist ein Methacrylat-Monomer, insbesondere ein Methacrylsäureester. Vorzugsweise handelt es sich bei dem Methacrylsäureester (a1) um einen monofunktionellen Methacrylsäureester. Dieser ist vorzugsweise hydrophob. Durch die Verwendung von hydrophoben monofunktionellen Methacrylsäureestern (a1) kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält. Vorzugsweise weist der monofunktionelle, hydrophobe Me- thacrylsäureester keine Carboxylgruppen, Hydroxylgruppen, Amidgruppen, Sulfonsäuregruppen, Sulfatgruppen, Phosphatgruppen oder Phosphonatgruppen auf.

Bei den Estern handelt es sich vorzugsweise um Alkylester. Unter Alkylester fallen erfindungsgemäß auch Cycloalkylester. Die Alkylester sind gemäß einer bevorzugten Ausführungsform Ester von Methacrylsäure mit Alkoholen, die 1 bis 20 Kohlenstoffatome, mehr bevorzugt 1 bis 10 Kohlenstoffatome, noch mehr bevorzugt 1 bis 6 Kohlenstoffatome und ganz besonders bevorzugt 1 bis 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Gemäß einer besonders bevorzugten Ausführungsform handelt es sich bei dem radikalisch polymerisierbaren Monomer (a1) um Methacrylsäuremethylester, Methacrylsäureethylester oder um eine Mischung aus diesen beiden Monomeren.

Gemäß einer weiteren bevorzugten Ausführungsform handelt es sich bei dem radikalisch polymerisierbaren Monomer (a1) nicht um einen von Bisphenol A abgeleiteten Methacrylsäureester.

Das erfindungsgemäß eingesetzte radikalisch polymerisierbare Monomer (a1) weist vorzugsweise eine Molmasse von weniger als 1.000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1.000 g/mol aufweist.

Das radikalisch polymerisierbare Monomer (a1) zeichnet sich vorzugsweise dadurch aus, dass eine wässrige Lösung des radikalisch polymerisierbaren Monomers (a1) einen pH-Wert im Bereich von 5 bis 9, vorzugsweise im Bereich von 5,5 bis 8,5, noch mehr bevorzugt im Bereich von 6 bis 8 und ganz besonders bevorzugt im Bereich von 6,5 bis 7,5 aufweist.

Paste A enthält vorzugsweise 15 bis 85 Gew.-%, mehr bevorzugt 20 bis 70 Gew.-%, noch mehr bevorzugt 25 bis 60 Gew.% und besonders bevorzugt 25 bis 50 Gew.-%, jeweils bezogen auf das Gesamtgewicht der Paste A, des wenigstens einen radikalisch polymerisierbaren Monomers (a1).

Paste A beinhaltet weiterhin als Komponente (a2) wenigstens ein Barbitursäurederivat als Polymerisationsinitiator, wobei das Barbitursäurederivat vorzugsweise ausgewählt ist aus der Gruppe bestehend aus 1-monosubstituierten Barbituraten, 5-monosubstituierten Barbituraten, 1,5-disubstituierten Barbituraten, 1,3,5-trisubstituierten Barbituraten und 1,3,5-tetrasubstituierten Barbituraten. Gemäß einer besonderen Ausgestaltung des erfindungsgemäßen Kits ist das Barbitursäurederivat ausgewählt ist aus der Gruppe bestehend aus 1,5-disubstituierten Barbituraten, 1,3,5-trisubstituierten Barbituraten und 1,3,5-tetrasubstituierten Barbituraten.

Gemäß einer bevorzugten Ausführungsform ist das Barbitursäurederivat (a2) in dem radikalisch polymerisierbaren Monomer (a1) löslich. Das Barbitursäurederivat (a2) ist in dem radikalisch polymerisierbaren Monomer (a1) löslich, wenn sich das Barbitursäurederivat (a3) in dem radikalisch polymerisierbaren Monomer (a1) zu wenigstens 1 g/l, vorzugsweise zu wenigstens 3 g/l, noch mehr bevorzugt zu wenigstens 5 g/l und ganz besonders bevorzugt zu wenigstens 10 g/l bei einer Temperatur von 25°C löst.

Die Art der Substituenten an der Barbitursäure ist nicht weiter eingeschränkt. Bei den Substituenten kann es sich beispielsweise um aliphatische oder aromatische Substituenten handeln. Hierbei können alkylische, cycloalkylische, allylische oder arylische Substituenten bevorzugt sein. Die Substituenten können auch Heteroatome tragen. Insbesondere kann es sich bei den Substituenten um Thiosubstituenten handeln. Demnach können 1,5- disubstituierte Thiobarbiturate oder 1,3,5-trisubstituierte Thiobarbiturate bevorzugt sein.

Gemäß einer bevorzugten Ausführungsform weisen die Substituenten jeweils eine Länge von 1 bis 10 Kohlenstoffatomen, mehr bevorzugt eine Länge von 1 bis 8 Kohlenstoffatomen und ganz besonders bevorzugt eine Länge im Bereich von 2 bis 7 Kohlenstoffatomen auf.

Erfindungsgemäß bevorzugt sind Barbiturate, die an Position 1 und an Position 5 jeweils einen Substituenten, an den Positionen 1, 3 und 5 jeweils einen Substituenten oder an den Positionen 1 und 3 jeweils einen Substituenten und an Position 5 zwei Substituenten tragen.

Gemäß einer weiteren bevorzugten Ausführungsform ist das BarbitursäureDerivat ein 1,5-disubstituiertes Barbiturat oder ein 1,3,5-trisubtituiertes Barbiturat.
Gemäß einer besonders bevorzugten Ausführungsform ist das Barbitursäurederivat (a2) aus der Gruppe ausgewählt, die aus 1-Cyclohexyl-5-ethyl-barbitursäure, 1-Phenyl-5-ethyl-barbitursäure und 1,3,5-Trimethyl-barbitursäure besteht.

Die Paste A beinhaltet das wenigstens eine Barbitursäurederivat (a2) vorzugsweise in einer Menge ein einem Bereich von 0,1 bis 10 Gew.-%, mehr bevorzugt in einem Bereich von 0,5 bis 8 Gew.-% und noch mehr bevorzugt in einem Bereich von 1 bis 5 Gew.-%, jeweils bezogen auf das Gesamtgewicht der Paste A.

Gemäß einer besonderen Ausführungsform des erfindungsgemäßen Kits liegt das Barbituräurederivat (a2) in der Paste A in einer Form vor, in der mindestens 50 Mol-%, besonders bevorzugt zu mindestens 75 Mol-% und am meisten bevorzugt mindestens 95 Mol-% des Barbitursäurederivates (a2) in der protonierten Säureform vorliegen. Insbesondere ist es in diesem Zusammenhang bevorzugt, dass das Barbitursäurederivat (a2) kein Salz des Barbitursäurederivates, insbesondere kein Calcium-Salz des Barbitursäurederivates, ist. Weiterhin ist es in diesem Zusammenhang bevorzugt, dass bei dieser besonderen Ausführungsform des erfindungsgemäßen Kits die Paste B weniger als 2 Gew.%, besonders bevorzugt weniger als 0,5 Gew.-%, noch mehr bevorzugt weniger als 0,1 Gew.-%, jeweils bezogen auf das Gesamtgewicht der Paste B, einer in dem wenigstens einen radikalisch polymerisierbaren Monomer (b1) löslichen organischen Säure, vorzugsweise einer Säure ausgewählt aus der Gruppe bestehend aus 2-Ethylhexansäure, Hexansäure, Heptansäure, Octansäure, Malonsäure, Monomeren mit Säurefunktionen, wie Sulfonsäure-, Phosphorsäure-, Phosphonsäure- und Carbonsäuregruppen, Essigsäure, Propionsäure, Pivalinsäure, Chloressigsäure, Methansulfonsäure und Phosphorsäure, beinhaltet. Am meisten bevorzugt ist es, dass die Paste B überhaupt keine dieser Säuren beinhaltet.
Paste B beinhaltet als Komponente (b1) ebenfalls ein radikalisch polymerisierbares Monomer, wobei es sich vorzugsweise um ein Monomer handelt, welches bei einer Temperatur von 25°C und einem Druck von 1.013 hPa flüssig ist. Das radikalisch polymerisierbare Monomer (b1) kann in einem Kit identisch sein mit dem radikalisch polymerisierbaren Monomer (a1) oder sich von diesem unterscheiden, wobei es bevorzugt ist, dass das radikalisch polymerisierbare Monomer (a1) und das radikalisch polymerisierbare Monomer (b1) identisch sind.

Das radikalisch polymerisierbare Monomer (b1) ist ein Methacrylat-Monomer, insbesondere ein Methacrylsäureester. Vorzugsweise handelt es sich bei dem Methacrylsäureester (b1) um einen monofunktionellen Methacrylsäureester. Dieser ist vorzugsweise hydrophob. Durch die Verwendung von hydrophoben monofunktionellen Methacrylsäureestern (b1) kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält. Vorzugsweise weist der monofunktionelle, hydrophobe Methacrylsäureester keine Carboxylgruppen, Hydroxylgruppen, Amidgruppen, Sulfonsäuregruppen, Sulfatgruppen, Phosphatgruppen oder Phosphonatgruppen auf.

Bei den Estern handelt es sich vorzugsweise um Alkylester. Unter Alkylester fallen erfindungsgemäß auch Cycloalkylester. Die Alkylester sind gemäß einer bevorzugten Ausführungsform Ester von Methacrylsäure mit Alkoholen, die 1 bis 20 Kohlenstoffatome, mehr bevorzugt 1 bis 10 Kohlenstoffatome, noch mehr bevorzugt 1 bis 6 Kohlenstoffatome und ganz besonders bevorzugt 1 bis 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Gemäß einer besonders bevorzugten Ausführungsform handelt es sich bei dem radikalisch polymerisierbaren Monomer (b1) um Methacrylsäuremethylester, Methacrylsäureethylester oder um eine Mischung aus diesen beiden Monomeren.

Gemäß einer weiteren bevorzugten Ausführungsform handelt es sich bei dem radikalisch polymerisierbaren Monomer (b1) nicht um einen von Bisphenol A abgeleiteten Methacrylsäureester.

Das erfindungsgemäß eingesetzte radikalisch polymerisierbare Monomer (b1) weist vorzugsweise eine Molmasse von weniger als 1.000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1.000 g/mol aufweist.

Das radikalisch polymerisierbare Monomer (b1) zeichnet sich vorzugsweise dadurch aus, dass eine wässrige Lösung des radikalisch polymerisierbaren Monomers (b1) einen pH-Wert im Bereich von 5 bis 9, vorzugsweise im Bereich von 5,5 bis 8,5, noch mehr bevorzugt im Bereich von 6 bis 8 und ganz besonders bevorzugt im Bereich von 6,5 bis 7,5 aufweist.

Paste B enthält vorzugsweise 15 bis 85 Gew.%, mehr bevorzugt 20 bis 70 Gew.-%, noch mehr bevorzugt 25 bis 60 Gew.-% und besonders bevorzugt 25 bis 50 Gew.-%, jeweils bezogen auf das Gesamtgewicht der Paste B, des wenigstens einen radikalisch polymerisierbaren Monomers (b1).

Paste B beinhaltet weiterhin als Komponente (b2) wenigstens eine basische Schwermetallverbindung, die aus der Gruppe bestehend aus Schwermetallsalzen und Schwermetallkomplexen ausgewählt ist, als Polymerisationsbeschleuniger, wobei es sich als besonders vorteilhaft erwiesen hat, wenn die wenigstens eine Schermetallverbindung (b2) in dem radikalisch polymerisierbaren Monomer (b1) schwer-, vorzugsweise sogar unlöslich ist. Als in dem radikalisch polymerisierbaren Monomer (b1) schwer- oder unlöslich gilt eine Schwermetallverbindung (b2), wenn sich weniger als 1 g/l, vorzugsweise weniger als 0,1 g/l, noch mehr bevorzugt weniger als 0,01 g/l, noch mehr bevorzugt weniger als 0,001 g/l, darüber hinaus bevorzugt weniger als 0,0001 g/l um am meisten bevorzugt überhaupt keine nennenswerten Mengen der Schwermetallverbindung (b2) bei einer Temperatur von 25°C in dem radikalisch polymerisierbaren Monomer (b1) lösen (die Schwermetallverbindung (b2) also in dem radikalisch polymerisierbaren Monomer (b1) unlöslich ist).

Unter Schwermetallverbindungen werden erfindungsgemäß Verbindungen von Metallen verstanden, die eine Dichte bei einer Temperatur von 20°C von wenigstens 3,5, vorzugsweise von wenigstens 5 aufweisen.

Gemäß einer bevorzugten Ausführungsform handelt es sich bei der Schwermetallverbindung (b2) um eine basische Schwermetallverbindung. Unter basischer Schwermetallverbindung wird eine Schwermetallverbindung verstanden, die in Wasser gelöst oder suspendiert einen pH-Wert von wenigstens 7,0, bevorzugt wenigstens 8 und noch mehr bevorzugt wenigstens 8,5 aufweist.

Gemäß einer ganz besonders bevorzugten Ausführungsform handelt es sich bei den Schwermetallverbindungen (b2) um Verbindungen von Metallen, die ihre Oxidationsstufe wechseln können. In diesem Zusammenhang erfindungsgemäß bevorzugt sind Verbindungen von Kup- fer (II), Eisen (II), Eisen (III), Mangan (II), Mangan (III), Cobalt (II) und Cobalt (III), wobei Kupfer (II)-Verbindungen ganz besonders bevorzugt sind.

Die erfindungsgemäßen Schwermetallverbindungen sind, sofern es sich um in dem radikalisch polymerisierbaren Monomer (b1) schwer- oder unlösliche Schermetallverbindungen handelt, vorzugsweise in der Lage, sich in Gegenwart der Barbitursäurederivate (a2) in eine in dem radikalisch polymerisierbaren Monomer (a1) bzw. (b1) lösliche Form umzuwandeln.

Erfindungsgemäß handelt es sich bei den Schwermetallverbindungen (b2) um Schwermetallsalze oder Schwermetallkomplexe.

Bei den Schwermetallsalzen (b2) handelt es sich vorzugsweise um Halogenide, Hydroxyde, Carbonate oder Carbonsäuresalze von Schwermetallen. Bevorzugte Schwermetallsalze sind Salze von Kupfer (II), Eisen (II), Eisen (III), Mangan (II), Mangan (III), Cobalt (II) und Cobalt (III).

Als Schwermetallverbindung (b2) kommen weiterhin Halogenidsalze in Betracht. Das Halogenidsalz kann vorzugsweise aus der Gruppe ausgewählt sein, die aus Chloriden und Bromiden von Schwermetallen besteht. Gemäß einer besonderen Ausführungsform handelt es sich bei dem Halogenidsalz um eine Verbindung, die aus der Gruppe ausgewählt ist, die aus Kupfer(II)-chlorid, Mangan(II)-chlorid, Eisen(II)-chlorid, Eisen(III)-chlorid, Kobalt(II)-chlorid und Kobalt(III)-chlorid, besteht.

Gemäß einer besonders bevorzugten Ausführungsform wird das Schwermetallsalz (b2) aus der Gruppe ausgewählt, die aus Kupfer(II)-hydroxid, basischem Kupfer(II)-carbonat, Kupfer(II)chlorid, Kupfer(II)-2-ethylhexanoat oder einer Mischung aus mindestens zwei davon, insbesondere einer Mischung aus Kupfer(II)-hydroxid und Kupfer(II)-carbonat, besteht.

Die Paste B beinhaltet die Schwerme- tallverbindung (b2) vorzugsweise in einer Menge in einem Bereich von 0,0005 bis 0,5 Gew.-%, mehr bevorzugt in einem Bereich von 0,001 bis 0,05 Gew.-% und ganz besonders bevorzugt in einem Bereich von 0,001 bis 0,01 Gew.-%, jeweils bezogen auf das Gesamtgewicht der Paste B.

Der erfindungsgemäß Kit ist gemäß Merkmal i) dadurch gekennzeichnet, dass Paste B weniger als 0,01 Gew.-%, besonders bevorzugt weniger als 0,001 Gew.-% und am meisten bevorzugt weniger als 0,0001 Gew.-%, jeweils bezogen auf das Gesamtgewicht der Paste B, eines Peroxids beinhaltet. Gemäß einer besonderen Ausführungsform des erfindungsgemäßen Kits beinhalten sowohl Paste A als auch Paste B weniger als 0,01 Gew.-%, besonders bevorzugt weniger als 0,001 Gew.-% und am meisten bevorzugt weniger als 0,0001 Gew.-%, jeweils bezogen auf das Gesamtgewicht der Paste A bzw. der Paste B, eines Peroxids. Am meisten bevorzugt ist es, dass weder die Paste A noch die Paste B ein Peroxid beinhalten. Unter einem Peroxid werden erfindungsgemäß Verbindungen verstanden, die wenigstens eine Peroxogruppe (-O-O-) enthalten.

Weiterhin ist der erfindungsgemäße Kit gemäß Merkmal ii) dadurch gekennzeichnet, dass mindestens eine der Pasten A und B als Komponente (a3) bzw. (b3) wenigstens einen in (a1) bzw. (b1) schwer- oder unlöslichen Füllstoff beinhaltet. Sofern eine der beiden Pasten einen schwer- oder unlöslichen Füllstoff beinhaltet und die andere Paste überhaupt keinen schwer- oder unlöslichen Füllstoff oder einen schwer- oder unlöslichen Füllstoff in einer im Vergleich zur Menge in der anderen Paste vernachlässigbaren Menge, so handelt es sich um einen sogenannten "asymmetrischen" Kit. Ist hingegen der schwer- oder unlösliche Füllstoff in beiden Pasten in etwa vergleichbarer Menge vorhanden, so handelt es sich um einen sogenannten "symmetrischen" Kit.

Bei dem schwer- oder unlöslichen Füllstoff (a3) (im Falle der Paste A) bzw. (b3) (im Falle der Paste B) handelt es sich um einen bei Raumtemperatur festen Stoff, der in der Lage ist, die Viskosität der aus den übrigen in Paste A bzw. Paste B enthaltenen Bestandteilen zusammen- gesetzten Mischung zu erhöhen. Der Füllstoff (a3) bzw. (b3) soll biokompatibel sein.

Gemäß einer bevorzugten Ausführungsform ist der schwer- oder unlösliche Füllstoff (a3) bzw. (b3) aus Polymeren, anorganischen Salzen, anorganischen Oxiden, Metallen und Metalllegierungen ausgewählt.

Der schwer- oder unlösliche Füllstoff (a3) bzw. (b3) ist vorzugsweise partikulär. Gemäß einer besonders bevorzugten Ausführungsform weist der schwer- oder unlösliche Füllstoff (a3) bzw. (b3) eine durchschnittliche Teilchengröße im Bereich von 10 nm bis 100 µm und besonders bevorzugt im Bereich von 100 nm bis 10 µm auf. Unter durchschnittlicher Teilchengröße wird vorliegend ein Größenbereich verstanden, der von wenigstens 90 Prozent der Teilchen eingenommen wird.

Im Rahmen der Erfindung werden unter dem Begriff Polymere sowohl Homopolymere als auch Copolymere zusammengefasst.

Bei dem als schwer- oder unlöslicher Füllstoff (a3) bzw. (b3) verwendbaren Polymer handelt es sich vorzugsweise um ein Polymer mit einer gewichtsmittleren Molmasse von wenigstens 150.000 g/mol. Die Angabe der Molmasse bezieht sich hierin auf die viskosimetrisch bestimmte Molmasse. Beispielsweise kann es sich bei dem Polymer um ein Polymer oder Copolymer eines Methacrylsäureesters handeln. Gemäß einer besonders bevorzugten Ausführungsform ist das wenigstens eine Polymer aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methylacrylat) und Poly(styren-co-methylmethacrylat) besteht. Das Polymer kann jedoch ebenso aus der Gruppe ausgewählt sein, die aus Polyethylen, Polypropylen oder Polybutadien besteht. Das Polymer kann zudem vernetzt oder unvernetzt sein, wobei vernetzte Polymere besonders bevorzugt sind. Die Vernetzung erfolgt dabei vorzugsweise mit einer difunktionellen Verbindung. Die difunktionelle Verbindung kann beispielsweise aus der Gruppe ausgewählt sein, die aus Alkylenglykoldimeth-acrylaten besteht. Als Vernetzter hat sich beispielsweise Ethylenglykol-dimethacrylat bewährt.

Das als schwer- oder unlöslicher Füllstoff (a3) bzw. (b3) verwendbare anorganische Salz kann ein im radikalisch polymerisierbaren Monomer (a1) bzw. (b1) lösliches oder unlösliches Salz sein. Vorzugsweise handelt es sich bei dem anorganischen Salz um ein Salz eines Elements, das aus der 2. Hauptgruppe des Periodensystems der Elemente ausgewählt wird. Gemäß einer bevorzugten Ausführungsform ist das anorganische Salz ein Salz von Calcium, Strontium oder Barium. Gemäß einer besonders bevorzugten Ausführungsform ist das anorganische Salz Kalziumsulfat, Bariumsulfat oder Kalziumcarbonat.

Bei dem als schwer- oder unlöslicher Füllstoff (a3) bzw. (b3) verwendbaren anorganischen Oxid kann es sich vorzugsweise um ein Metalloxid handeln. Gemäß einer bevorzugten Ausführungsform ist das anorganische Oxid ein Oxid der Übergangsmetalle. Gemäß einer besonders bevorzugten Ausführungsform handelt es sich bei dem anorganischen Oxid um Titandioxid oder Zirkoniumdioxid.

Bei dem als schwer- oder unlöslicher Füllstoff (a3) bzw. (b3) verwendbaren Metall kann es sich beispielsweise um ein Übergangsmetall handeln. Gemäß einer bevorzugten Ausführungsform ist das Metall Tantal oder Wolfram.

Die als schwer- oder unlöslicher Füllstoff (a3) bzw. (b3) verwendbare Metalllegierung ist eine Legierung von wenigstens zwei Metallen. Vorzugsweise enthält die Legierung wenigstens ein Übergangsmetall. Gemäß einer besonders bevorzugten Ausführungsform weist die Legierung wenigstens Tantal oder Wolfram auf. Bei der Legierung kann es sich auch um eine Legierung aus Tantal und Wolfram handeln.

Der Füllstoff (a3) bzw. (b3) ist in dem radikalisch polymerisierbaren Monomer (a1) bzw. (b1) schwer- oder unlöslich. Erfindungsgemäß ist der Füllstoff (a3) bzw. (b3) in dem radikalisch polymerisierbaren Monomer (a1) bzw. (b1) schwer- oder unlöslich, wenn der Füllstoff (a3) bzw. (b3) bei einer Temperatur von 25°C eine Löslichkeit im radikalisch polymerisierbaren Monomer (a1) bzw. (b1) von weniger als 50 g/l, vorzugsweise weniger als 25 g/l, mehr bevorzugt weniger als 10 g/l, noch mehr bevorzugt weniger als 5 g/l und noch mehr bevorzugt weniger als 0,5 g/l aufweist, wobei es am meisten bevorzugt ist, dass sich der schwer- oder unlösliche Füllstoff (a3) bzw. (b3) in dem radikalisch polymerisierbaren Monomer (a1) bzw. (b1) überhaupt nicht oder aber allenfalls in vernachlässigbar geringer Menge löst.

Erfindungsgemäß besonders bevorzugt ist es, dass das wenigstens eine in (a1) bzw. (b1) schwer- oder unlösliche Polymer ausgewählt ist aus der Gruppe bestehend aus vernetztem Poly(methylmethacrylat-co-methylacrylat), vernetztem Poly(methylmeth-acrylat) und einer Mischung dieser beiden Polymere.

Weiterhin ist der erfindungsgemäße Kit gemäß Merkmal iii) dadurch gekennzeichnet, dass mindestens einer der Pasten A und B, besonders bevorzugt die Paste B und insbesondere ausschließlich die Paste B, als Komponente (a4) bzw. (b4) wenigstens ein anorganisches Halogenid-Salz beinhaltet, wobei es sich als vorteilhaft erwiesen hat, wenn dieses anorganische Halogenid-Salz (a4) bzw. (b4) in dem radikalisch polymerisierbaren Monomer (a1) bzw. (b1) zumindest teilweise löslich ist. Als in dem radikalisch polymerisierbaren Monomer (a1) bzw. (b1) zumindest teilweise löslich wird dabei vorzugsweise ein anorganische Halogenid-Salz (a4) bzw. (b4) verstanden, das eine Löslichkeit von wenigstens 0,001 g/l, mehr bevorzugt von wenigstens 0,01 g/l, noch mehr bevorzugt von wenigstens 0,1 g/l und noch mehr bevorzugt von wenigstens 1 g/l in dem polymerisierbaren Monomer, das in der Paste, in der auch das Halogenid-Salz enthalten ist, aufweist. Vorzugsweise handelt es sich bei dem wenigstens einen anorganischen Halogenid-Salz (a4) bzw. (b4) um ein Halogenid-Salz aus mindestens einem Halogenid-Anion und einem Metall-Kation, wobei als Halogenid-Anion F⁻, Cl⁻ und Br⁻ in Betracht kommen und Cl⁻ ganz besonders bevorzugt ist. Vorzugswiese ist das wenigstens eine anorganische Halogenid-Salz (a4) bzw. (b4) ausgewählt aus der Gruppe bestehend aus einem Alkalihalogenid, einem Erdalkalihalogenid, und einer Mischung aus mindestens zwei dieser Halogenid-Salze. Zu den besonders bevorzugten HalogenidSalzen gehören Lithiumchlorid, Natriumchlorid, Kaliumchlorid, Kalziumchlorid und Magnesiumchlorid. Erfindungsgemäß ganz besonders bevorzugt als anorganisches Halogenid-Salz (a4) bzw. (b₄) ist Lithiumchlorid.

Gemäß einer ersten besonderen Ausgestaltung des erfindungsgemäßen Kits beinhaltet die Paste B nicht 40 mg Lithiumchlorid pro 36,099 g der Paste B. Dementsprechend beträgt gemäß dieser Ausführungsform der Anteil von Lithiumchlorid nicht 0,1108 Gewichtsprozent, bezogen auf das Gesamtgewicht von Paste B.

Gemäß einer zweiten besonderen Ausgestaltung des erfindungsgemäßen Kits beinhaltet die Paste B nicht 2 mg Kupfer(II)-hydroxid pro 36,099 g der Paste B. Dementsprechend beträgt gemäß dieser Ausführungsform der Anteil von Kupfer(II)-hydroxid nicht 0,00554 Gewichtsprozent, bezogen auf das Gesamtgewicht von Paste B. Insbesondere beinhaltet Paste B bei dieser zweiten besonderen Ausgestaltung des erfindungsgemäßen Kits mehr als 0,00554 Gew.-%, besonders bevorzugt mehr als 0,0075 Gew.-% und noch mehr bevorzugt mehr als 0,01 Gew.-% der Schwermetallverbindung (b2), jeweils bezogen auf das Gesamtgewicht der Paste B.

Gemäß einer dritten besonderen Ausgestaltung des erfindungsgemäßen Kits beinhaltet die Paste B die Schwermetallverbindung (b2) und das anorganische Halogenid-Salz (b4) nicht in einem relativen Massenverhältnis Schwermetallverbindung (b2) : anorganisches Halogenid-Salz (b4) von 1 : 20. Insbesondere beinhaltet Paste B bei dieser dritten besonderen Ausgestaltung des erfindungsgemäßen Kits die Schwermetallverbindung (b2) und das anorganische Halogenid-Salz (b4) in einem relativen Massenverhältnis Schwermetallverbindung (b2): anorganische Halogenid-Salz (b4) von mehr als 1 : 20, vorzugsweise von mehr als 1 : 10.

In diesem Zusammenhang ist es weiterhin bevorzugt, dass die Paste A oder die Paste B oder die Paste A und die Paste B das mindestens eine anorganische Halogenid-Salz (a4) bzw. (b4) in einer Menge in einem Bereich von 0,001 bis 7,5 Gew.-%, besonders bevorzugt in einem Bereich von 0,01 bis 5 Gew.-%, noch mehr bevorzugt in einem Bereich von 0,1 bis 2,5 Gew.-% und am meisten bevorzugt in einem Bereich von 0,5 bis 1,5 Gew.-%, jeweils bezogen auf das Gesamtgewicht der Paste A bzw. B, beinhalten.

Weiterhin kann erfindungsgemäß die Paste A, die Paste B oder die Paste A und die Paste B ein in (a1) bzw. (b1) lösliches Polymer (a5) bzw. (b5) beinhalten. Dieses Polymer (a5) bzw. (b5) ist erfindungsgemäß in dem polymerisierbaren Monomer, das in der Paste enthalten ist, in der auch das lösliche Polymer enthalten ist, löslich, wenn sich das Polymer in diesem polymerisierbaren Monomer zu wenigstens 10 g/l, vorzugsweise zu wenigstens 25 g/l, mehr bevorzugt zu wenigstens 50 g/l und ganz besonders bevorzugt zu wenigstens 100 g/l, löst. Das in dem polymerisierbaren Monomer (a1) bzw. (b1) lösliche Polymer (a5) bzw. (b5) kann ein Homopolymer oder ein Copolymer sein. Bei diesem Polymer (a5) bzw. (b5) handelt es sich vorzugsweise um ein Polymer mit einer gewichtsmittleren Molmasse von wenigstens 150.000 g/mol. Beispielsweise kann es sich bei dem Polymer (a5) bzw. (b5) um ein Polymer oder Copolymer eines Methacrylsäureesters handeln. Gemäß einer besonders bevorzugten Ausführungsform ist das wenigstens eine lösliche Polymer (a5) bzw. (b5) aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Po-ly(methylmethacrylat-co- methylacrylat), Poly(styren-co-methylmethacrylat) und einer Mischung aus mindestens zwei dieser Polymere besteht.

Die Menge des in dem radikalisch polymerisierbaren Monomer (a1) bzw. (b1) löslichen Polymers (a5) bzw. (b5) in der Paste, die dieses Polymer enthält, hängt davon ab, ob die entsprechende Paste einen in dem radikalisch polymerisierbaren Monomer (a1) bzw. (b1) unlöslichen Füllstoff (a3) bzw. (b3) enthält. Üblicherweise liegt die Menge des in dem radikalisch polymerisierbaren Monomer (a1) bzw. (b1) löslichen Polymers (a5) bzw. (b5) in der Paste, die dieses Polymer enthält, in einem Bereich von 1 bis 85 Gew.-%, bezogen auf das Gesamtgewicht der Paste, welche dieses lösliche Polymer enthält.

Neben den vorstehend erläuterten Bestandteilen können die Pasten A und B weitere Bestandteile aufweisen. Diese weiteren Bestandteile können jeweils entweder in Paste A, in Paste B oder in der Paste A und der Paste B enthalten sein.

Gemäß einer bevorzugten Ausführungsform ist in wenigstens einer der Pasten A und B wenigstens ein Röntgenopaker enthalten. Beim Röntgenopaker kann es sich um einen fachüblichen Röntgenopaker handeln. Geeignete Röntgenopaker können in dem radikalisch polymerisierbaren Monomer (a1) oder dem radikalisch polymerisierbaren Monomer (b1) löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen besteht. Diese Röntgenopaker weisen vorzugsweise einen mittleren Teilchendurchmesser im Bereich von 10 nm bis 500 µm auf. Als Röntgenopaker kommen ferner auch Ester der 3,5-Bis(acetamido)-2,4,6-triiodbenzoesäure, Gadolinium-Verbindungen, wie Gadolinium-Chelat mit den Estern der 1,4,7,10-Tetraazacyclododecan-1,4,7,10-tetraessigsäure (DOTA), in Betracht.

Gemäß einer weiteren bevorzugten Ausführungsform ist in wenigstens einer der Pasten A und B wenigstens ein Farbstoff enthalten. Der Farbstoff kann ein fachüblicher Farbstoff und vorzugsweise ein Lebensmittelfarbstoff sein. Ferner kann der Farbstoff in dem wenigstens einen radikalisch polymerisierbaren Monomer (a1) oder in dem wenigstens einen radikalisch polymerisierbaren Monomer (a2) löslich oder unlöslich sein. Gemäß einer besonders bevorzugten Ausführungsform wird der Farbstoff aus der Gruppe ausgewählt, die aus E101, E104, E132, E141 (Chlorophyllin), E142, Riboflavin und Lissamingrün besteht. Unter den Begriff Farbstoff fallen erfindungsgemäß auch Farblacke, wie zum Beispiel Farblack Grün, das Aluminiumsalz einer Mischung aus E104 und E132.

Gemäß einer weiteren bevorzugten Ausführungsform ist in wenigstens einer der Pasten A und B wenigstens ein pharmazeutischer Wirkstoff enthalten. Der wenigstens eine pharmazeutische Wirkstoff kann in wenigstens einer der Pasten A und B in gelöster oder suspendierter Form enthalten sein.

Der pharmazeutische Wirkstoff kann vorzugsweise aus der Gruppe ausgewählt sein, die aus Antibiotika, Aniphlogistika, Steroiden, Hormonen, Wachstumsfaktoren, Bisphosphonaten, Cytostatika und Genvektoren besteht. Gemäß einer besonders bevorzugten Ausführungsform handelt es sich bei dem wenigstens einen pharmazeutischen Wirkstoff um ein Antibiotikum.

Das wenigstens eine Antibiotikum ist vorzugsweise aus der Gruppe ausgewählt, die aus Aminoglykosid-Antibiotika, Glykopeptid-Antibiotika, Lincosamid-Antibiotika, Gyrasehemmern, Carbapenemen, cyclischen Lipopeptiden, Glycylcyclinen, Oxazolidonen und Polypeptidantibiotika besteht.

Gemäß einer besonders bevorzugten Ausführungsform handelt es sich bei dem wenigstens einen Antibiotikum um ein Mitglied, das aus der Gruppe ausgewählt ist, die aus Gentamicin, Tobramycin, Amikacin, Vancomycin, Teicoplanin, Dalbavancin, Lincosamin, Clindamycin, Moxifloxacin, Levofloxacin, Ofloxacin, Ciprofloxacin, Doripenem, Meropenem, Tigecyclin, Linezolid, Eperezolid, Ramoplanin, Metronidazol, Tinidazol, Omidazol und Colistin sowie Salzen und Estern davon besteht.

Demnach kann das wenigstens eine Antibiotikum aus der Gruppe ausgewählt sein, die aus Gentamicinsulfat, Gentamicinhydrochlorid, Amikacinsulfat, Amikacinhydrochlorid, Tobramycinsulfat, Tobramycinhydrochlorid, Clindamycinhydrochlorid, Lincosaminhydrochlorid und Moxifloxacin besteht.

Das wenigstens eine Antiphlogistikum ist vorzugsweise aus der Gruppe ausgewählt, die aus nicht-steroidalen Antiphlogistika und Glukokortikoiden besteht. Gemäß einer besonders bevorzugten Ausführungsform ist das wenigstens eine Antiphlogistikum aus der Gruppe ausgewählt, die aus Acetylsalicylsäure, Ibuprofen, Diclofenac, Ketoprofen, Dexamethason, Prednison, Hydrocortison, Hydrocortisonacetat und Fluticason besteht.

Das wenigstens eine Hormon ist vorzugsweise aus der Gruppe ausgewählt, die aus Serotonin, Somatotropin, Testosteron und Östrogen besteht.

Der wenigstens eine Wachstumsfaktor ist vorzugsweise aus der Gruppe ausgewählt, die aus dem *Fibroblast Growth Factor* (FGF), dem *Transforming Growth Factor* (TGF), dem *Platelet Derived Growth Factor* (PDGF), dem Epidermalen Wachstumsfaktor (EGF), dem *Vascular Endothelial Growth Factor* (VEGF), insulinähnlichen Wachstumsfaktoren (IGF), dem *Hepatocyte Growth Factor* (HGF), dem *Bone Morphogenetic Protein* (BMP), Interleukin-1B, Interleukin 8 und dem Nervenwachstumsfaktor besteht.

Das wenigstens eine Cytostatikum ist vorzugsweise aus der Gruppe ausgewählt, die aus Alkylantien, Platinanaloga, Interkalantien, Mitosehemmern, Taxanen, Topoisomerasehemmern und Antimetaboliten besteht.

Das wenigstens eine Bisphosphonat ist vorzugsweise aus der Gruppe ausgewählt, die aus Zoledronat und Aledronat besteht.

Gemäß einer weiteren bevorzugten Ausführungsform ist in wenigstens einer der Pasten A und B wenigstens ein biokompatibles Elastomer enthalten. Das biokompatible Elastomer ist vorzugsweise partikulär. Vorzugsweise ist das biokompatible Elastomer in dem wenigstens einen radikalisch polymerisierbaren Monomer (a1) oder in dem wenigstens einen radikalisch polymerisierbaren Monomer (b1) löslich. Als besonders geeignet hat sich die Verwendung von Polybutadien als biokompatibles Elastomer erwiesen.

Gemäß einer weiteren bevorzugten Ausführungsform enthält wenigstens eine der Pasten A und B wenigstens ein Monomer mit Haftgruppen. Bei der Haftgruppe kann es sich beispielsweise um eine Amidgruppe handeln. Das Monomer mit Haftgruppe kann daher zum Beispiel Methacrylsäureamid sein. Durch die Verwendung von wenigstens einem Monomer mit Haftgruppen kann die Anbindung des Knochenzementes an Gelenkendoprothesen gezielt beeinflusst werden.

Gemäß einer weiteren bevorzugten Ausführungsform ist in wenigstens einer der Pasten A und B wenigstens ein Stabilisator enthalten. Der Stabilisator soll geeignet sein, eine Spontanpolymerisation der in den Pasten A und B enthaltenen radikalisch polymerisierbaren Monomere (a1) bzw. (b1) zu verhindern. Ferner soll der Stabilisator keine störenden Wechselwirkungen mit den anderen in den Pasten enthaltenen Bestandteilen zeigen. Derartige Stabilisatoren sind aus dem Stand der Technik bekannt. Gemäß einer bevorzugten Ausführungsform handelt es sich bei dem Stabilisator um 2,6-Di-tert-butyl-4-methylphenol und/oder 2,6-Di-tert-butylphenol.

Gemäß einer ersten besonderen Ausgestaltung des erfindungsgemäßen Kits handelt es sich bei dem Kit um einen "asymmetrischen" Kit. In diesem Zusammenhang ist es bevorzugt, dass die Paste A 20 bis 70 Gew.-%, besonders bevorzugt 25 bis 60 Gew.%, noch mehr bevorzugt 30 bis 55 Gew.% und am meisten bevorzugt 34 bis 47 Gew.-%, jeweils bezogen auf das Gesamtgewicht der Paste A, des in (a1) unlöslichen Füllstoffs (a3) und Paste B weniger als 5 Gew.-%, besonders bevorzugt weniger als 1 Gew.-%, noch mehr bevorzugt weniger als 0,1 Gew.-% und darüber hinaus bevorzugt weniger als 0,01 Gew.-%, jeweils bezogen auf das Gesamtgewicht der Paste B, des in (b1) unlöslichen Füllstoffs (b3) beinhaltet, wobei es am meisten bevorzugt ist, dass die Paste B überhaupt keinen in (b1) unlöslichen Füllstoff (b3) beinhaltet.

Weiterhin ist es im Zusammenhang mit dieser ersten besonderen Ausgestaltung des erfindungsgemäßen Kits bevorzugt, dass Paste A ein in (a1) lösliches Polymer (a5) in einer Menge in einem Bereich von 1 bis 25 Gew.-%, besonders bevorzugt in einem Bereich von 2 bis 20 Gew.-%, noch mehr bevorzugt in einem Bereich von 2 bis 18 Gew.-% und am meisten bevorzugt in einem Bereich von 3 bis 16 Gew.-%, jeweils bezogen auf das Gesamtgewicht der Paste A, und Paste B ein in (b1) lösliches Polymer (b5) in einer Menge in einem Bereich von 25 bis 85 Gew.-%, besonders bevorzugt in einem Bereich von 35 bis 85 Gew.-%, noch mehr bevorzugt in einem Bereich von 40 bis 80 Gew.-% und am meisten bevorzugt in einem Bereich von 50 bis 75 Gew.-%, jeweils bezogen auf das Gesamtgewicht der Paste B, beinhaltet.

Weiterhin ist es im Zusammenhang mit dieser ersten besonderen Ausgestaltung des erfindungsgemäßen Kits bevorzugt, dass das Gewichtsverhältnis von in (b1) unlöslichem Füllstoff (b3) zu dem wenigstens einen in (b1) löslichen Polymer (b5) höchstens 0,2, besonders bevorzugt höchstens 0,15, noch mehr bevorzugt höchstens 0,1, nicht mehr bevorzugt höchstens 0,05, besonders bevorzugt höchstens 0,02 und ganz besonders bevorzugt 0 beträgt.

Gemäß einer zweiten besonderen Ausgestaltung des erfindungsgemäßen Kits handelt es sich bei dem Kit um einen "symmetrischen" Kit. In diesem Zusammenhang ist es bevorzugt, dass die Paste A 15 bis 85 Gew.-%, besonders bevorzugt 15 bis 80 Gew.-% und noch mehr bevorzugt 20 bis 75 Gew.-%, jeweils bezogen auf das Gesamtgewicht der Paste A, des in (a1) unlöslichen Füllstoffs (a3) und Paste B 15 bis 85 Gew.-%, besonders bevorzugt 15 bis 80 Gew.-% und noch mehr bevorzugt 20 bis 75 Gew.-%, jeweils be- zogen auf das Gesamtgewicht der Paste B, des in (b1) unlöslichen Füllstoffs (b3) beinhaltet.

Weiterhin ist es im Zusammenhang mit dieser zweiten besonderen Ausgestaltung des erfindungsgemäßen Kits bevorzugt, dass Paste A ein in (a1) lösliches Polymer (a5) in einer Menge in einem Bereich von 5 bis 50 Gew.-%, besonders bevorzugt in einem Bereich von 10 bis 40 Gew.-% und noch mehr bevorzugt in einem Bereich von 20 bis 30 Gew.-%, jeweils bezogen auf das Gesamtgewicht der Paste A, und/oder Paste B ein in (b1) lösliches Polymer (b5) in einer Menge in einem Bereich von 5 bis 50 Gew.-%, besonders bevorzugt in einem Bereich von 10 bis 40 Gew.-% und noch mehr bevorzugt in einem Bereich von 20 bis 30 Gew.-%, bezogen auf das Gesamtgewicht der Paste B, beinhaltet.

Erfindungsgemäß dient der Kit, der wenigstens die Pasten A und B enthält, zur Herstellung von Knochenzement.

Dazu werden die wenigstens zwei Pasten A und B miteinander vermischt, wobei wiederum eine Paste, Paste C, erhalten wird.

Das Mischungsverhältnis beträgt vorzugsweise 0,5 bis 1,5 Gewichtsteile an Paste A und 0,5 bis 1,5 Gewichtsteile an Paste B. Gemäß einer besonders bevorzugten Ausführungsform beträgt der Anteil der Paste A 30 bis 70 Gew.-% und der Anteil der Paste B 30 bis 70 Gew.-%, jeweils bezogen auf das Gesamtgewicht der Pasten A und B.

Das Mischen kann mit üblichen Mischgeräten, beispielsweise einem statischen Mischer oder einem dynamischen Mischer erfolgen.

Das Mischen kann unter Vakuum erfolgen. Die Verwendung des erfindungsgemäßen Initiatorsystems erlaubt jedoch auch das vakuumfreie Mischen der Paste A und B ohne Beeinträchtigung der Eigenschaften des Knochenzements.

Nach dem Vermischen der Pasten des Kits ist die letztlich erhaltene Paste C nach der Norm ISO 5833 klebfrei und kann sofort verarbeitet werden.

Der aus der Paste C durch Aushärtung entstehende Knochenzement erreicht etwa sechs bis acht Minuten nach dem Vermischen der im Kit enthaltenen Pasten eine hohe Festigkeit.

Der erfindungsgemäße Kit kann gemäß einer bevorzugten Ausführungsform zur mechanischen Fixierung von Gelenkendoprothesen, zur Abdeckung von Schädeldefekten, zur Auffüllung von Knochenkavitäten, zur Femuroplastie, zur Vertebroplastie, zur Kyphoplastie, zur Herstellung von Spacern und zur Herstellung von Trägermaterialien für die lokale Antibiotika-Therapie verwendet werden.

Unter dem Begriff "*Spacer*" werden dabei erfindungsgemäß Implantate verstanden, die temporär im Rahmen des zweizeitigen Prothesenwechsels bei septischen Revisionen als Platzhalter verwendet werden.

Trägermaterialien für die lokale Antibiotika-Therapie können als Kugeln oder kugelähnliche Körper oder als bohnenförmige Körper ausgebildet sein. Daneben ist es auch möglich, stabförmige oder scheibenförmige Trägermaterialien herzustellen, die den aus dem erfindungsgemäßen Kit hergestellten Knochenzement hergestellten Knochenzement erhalten. Weiterhin können die Trägermaterialien auch auf resorbierbarem oder nicht resorbierbarem Fadenmaterial perlschnurförmig aufgereiht sein.

Die vorstehend beschriebenen erfindungsgemäßen Verwendungen von Knochenzement sind aus der Literatur bekannt und dort mannigfach beschrieben.

Erfindungsgemäß wird der Kit für die vorstehend beschrieben Verwendungen eingesetzt, indem vorzugsweise aus den im Kit enthaltenen Pasten durch Vermischen eine Paste hergestellt wird, die analog zu den aus dem Stand der Technik bekannten Pasten für die beschriebenen Verwendungen eingesetzt wird.

Die Erfindung wird durch die nachstehend beschriebenen Beispiel erläutert, die jedoch die Erfindung nicht einschränken.

### AUSFÜHRUNGSBEISPIELE

Die bei den Beispielen A1-12 und B1-14 eingesetzten Chemikalien waren in pa.-Reinheit und wurden vom Chemikaliengroßhandel bezogen.

Es wurde weiterhin ein Polymethylmethacrylat-co-methylacrylat mit einer Molmasse von < 500000 g/mol als lösliches Polymer (a5) bzw. (b5) verwendet. Außerdem kam ein mit Ethylenglykoldimethacrylat vernetztes Polymethylmethacrylat der Siebfraktion < 70 µm als unlöslicher Füllstoff (a3) bzw. (b3) zur Anwendung. Es wurde weiterhin Gentamicinsulfat (Fujian Fukang Ltd.) mit einem Aktivitätskoeffizienten von AK = 620 eingesetzt.

Die jeweiligen Pasten A1 bis A12 und B1 bis B14 wurden so hergestellt, dass zuerst in einem inerten Kunststoffgefäß das Methylmethacrylat (Komponente (a1) bzw. (b1) eingewogen wurde. Die gesamten Feststoffkomponenten wurden in eine separate Plastikweithalsflasche mit Schraubdeckel eingewogen. Dazu wurden jeweils drei Porzellankugeln gegeben. Danach wurden die Gemische mit Hilfe eines Taumelmischers 1 Stunde homogenisiert. Die homogenisierten Pulverkomponenten wurden dem vorgelegten Methylmethacrylat zugesetzt. Die entstandenen Gemische wurden dann intensiv vermischt. Es bildeten sich Pasten, die vor der Vermischung bei Raumtemperatur über Nacht gelagert wurden, bis der Endzustand der Quellung erreicht wurde und sich streichfähige Pasten gebildet hatten.

**Paste A**

| | Zusammensetzung [g] | | | | | |
|---|---|---|---|---|---|---|
| | Beispiele | | | | | |
| | A1 | A2 | A3 | A4 | A5 | A6 |
| Lösliches Polymethylmethacrylat (a5) | 6,0 | 6,0 | 6,0 | 6,0 | 6,0 | 6,0 |
| Partikuläres Polymethylmethacrylat (a3) | 16,5 | 17,0 | 14,0 | 15,0 | 14,5 | 15,5 |
| Methylmethacrylat (a1) | 19,0 | 19,0 | 19,0 | 19,0 | 19,0 | 19,0 |
| 1-Cyclohexyl-5-ethylbarbitursäure (a2) | 2,0 | 1,5 | 2,0 | 2,0 | 1,5 | 1,5 |
| Gentamicinsulfat | - | - | 2,0 | 1,0 | 2,0 | 1,0 |
| Aerosil 300 | 0,4 | 0,4 | 0,4 | 0,4 | 0,4 | 0,4 |

**Paste A**

| | Zusammensetzung [g] | | | | | |
|---|---|---|---|---|---|---|
| | Beispiele | | | | | |
| | A7 | A8 | A9 | A10 | A11 | A12 |
| Lösliches Polymethylmethacrylat (a5) | 6,0 | 6,0 | 6,0 | 6,0 | 11,5 | 11,5 |
| Partikuläres Polymethylmethacrylat (a3) | 13,0 | 13,0 | 11,0 | 12,0 | 8,0 | 8,5 |
| Methylmethacrylat (a1) | 17,0 | 17,0 | 17,0 | 17,0 | 19,0 | 10,0 |
| Zirkoniumdioxid | 5,0 | | 5,0 | - | 2,5 | - |
| Bariumsulfat | - | 5,0 | - | 5,0 | - | 2,5 |
| 1-Cyclohexyl-5-ethylbarbitursäure (a2) | 2,0 | 2,0 | 2,0 | 2,0 | 2,0 | 1,5 |
| Gentamicinsulfat | - | - | 2,0 | 1,0 g | - | 0,5 |
| Aerosil 300 | 0,4 | 0,4 | 0,4 | 0,4 | 0,4 | 0,4 |

**Paste B**

| | Zusammensetzung [g] | | | | | |
|---|---|---|---|---|---|---|
| | Beispiele | | | | | |
| | B1 | B2 | B3 | B4 | B5 | B6 |
| Lösliches Polymethylmethacrylat (b5) | 17,0 | 17,0 | 17,0 | 17,0 | 17,0 | 17,0 |
| Methylmethacrylat (b1) | 21,0 | 21,0 | 21,0 | 21,0 | 21,0 | 21,0 |
| Zirkoniumdioxid | 5,0 | 5,0 | 5,0 | - | - | - |
| Bariumsulfat | - | - | - | 5,0 | 5,0 | 5,0 |
| Kupfer(II)hydroxid (b2) | 0,002 | 0,004 | 0,008 | 0,002 | 0,004 | 0,008 |
| Lithiumchlorid (b4) | 0,04 | 0,08 | 0,08 | 0,04 | 0,08 | 0,08 |

**Paste B**

| | Zusammensetzung [g] | | | | | |
|---|---|---|---|---|---|---|
| | Beispiele | | | | | |
| | B7 | B8 | B9 | B10 | B11 | B12 |
| Lösliches Polymethylmethacrylat (b5) | 17,0 | 17,0 | 17,0 | 17,0 | 17,0 | 17,0 |
| Methylmethacrylat (b1) | 22,0 | 22,0 | 22,0 | 22,0 | 22,0 | 22,0 |
| Zirkoniumdioxid | 5,0 | 5,0 | 5,0 | - | - | - |
| Bariumsulfat | - | - | - | 5,0 | 5,0 | 5,0 |
| Kupfer(II)hydroxid-Kupfer(II)carbonat (b2) | 0,004 | 0,008 | 0,016 | 0,004 | 0,008 | 0,016 |
| Lithiumchlorid (b4) | 0,04 | 0,08 | 0,08 | 0,04 | 0,08 | 0,08 |

**Paste B**

| | Zusammensetzung [g] | |
|---|---|---|
| | Beispiele | |
| | B13 | B14 |
| Lösliches Polymethylmethacrylat (b5) | 11,5 | 11,5 |
| Partikuläres Polymethylmethacrylat (b3) | 10,0 | 10,0 |
| Methylmethacrylat (b1) | 19,0 | 19,0 |
| Zirkoniumdioxid | 2,5 | - |
| Bariumsulfat | - | 2,5 |
| Kupfer(II)hydroxid (b2) | 0,002 | 0,004 |
| Lithiumchlorid (b4) | 0,04 | 0,08 |

Die Pasten A1 bis A12 wurden jeweils mit den Pasten B1 bis B12 im Gewichtsverhältnis 1 : 1 vermischt. Die gemischten Pasten waren sofort klebfrei. Der farblose Zementteig konnte 3,0 bis 7,0 Minuten verarbeitet werden. Die exotherme Aushärtung fand in den nachfolgenden 2 bis 4 Minuten statt.

Zusätzlich wurden die Pasten A11 und A12 mit den Pasten B13 und B14 ebenfalls im Gewichtsverhältnis 1 : 1 miteinander verknetet. Der jeweils klebfreie, farblose Zementteig konnte ca. 4 Minuten verarbeitet werden und härtete dann nach weiteren 3 bis 4 Minuten aus.

## Patentansprüche

1. Kit zur Herstellung eines Knochenzementes, aufweisend eine Paste A und eine Paste B, wobei
(a) Paste A
(a1) wenigstens ein Methacrylat-Monomer, und
(a2) als Polymerisationsinitiator wenigstens ein Barbitursäurederivat beinhaltet;
(b) Paste B
(b1) wenigstens ein Methacrylat-Monomer, und
(b2) als Polymerisationsbeschleuniger wenigstens eine basische Schwermetallverbindung, die aus der Gruppe bestehend aus Schwermetallsalzen und Schwermetallkomplexen ausgewählt ist, beinhaltet;
und wobei
i) Paste B weniger als 0,01 Gew.-%, bezogen auf das Gesamtgewicht der Paste B, eines Peroxids beinhaltet,
ii) mindestens eine der Pasten A und B als Komponente (a3) bzw. (b3) wenigstens einen in (a1) bzw. (b1) schwer- oder unlöslichen Füllstoff beinhaltet,
iii) mindestens eine der Pasten A und B als Komponente (a4) bzw. (b4) wenigstens ein anorganisches Halogenid-Salz beinhaltet, wobei
das wenigstens eine anorganische Halogenid-Salz (a4) bzw. (b4) ausgewählt ist aus der Gruppe bestehend aus einem Alkalihalogenid, einem Erdalkalihalogenid, und einer Mischung aus mindestens zwei dieser anorganischen Halogenid-Salze, und
iv) wobei Paste B weniger als 2 Gew.-% bezogen auf das Gesamtgewicht der Paste B einer organischen Säure enthält, die in Monomer (b1) löslich ist.

2. Kit nach Anspruch 1, wobei die Paste A und die Paste B das wenigstens eine radikalisch polymerisierbare Monomer (a1) bzw. (b1) in einer Menge in einem Bereich von 15 bis 85 Gew.-%, jeweils bezogen auf das Gesamtgewicht der Paste A bzw. der Paste B, beinhalten.

3. Kit nach Anspruch 1 oder 2, wobei das Barbitursäurederivat (a2) ausgewählt ist aus der Gruppe bestehend aus 1-monsubstituierten Barbituraten, 5-monosubstituierten Barbituraten, 1,5-disubstituierten Barbituraten, 1,3,5-trisubstituierten Barbituraten und 1,3,5-tetrasubstituierten Barbituraten.

4. Kit nach einem der vorhergehenden Ansprüche, wobei das wenigstens eine Barbitursäurederivat (a2) ausgewählt ist aus der Gruppe bestehend aus 1-Cyclohexyl-5-ethyl-barbitursäure, 1-Phenyl-5-ethyl-barbitursäure und 1,3,5-Trimethyl-barbitursäure.

5. Kit nach einem der vorhergehenden Ansprüche, wobei die Paste A das wenigstens eine Barbitursäurederivat (a2) in einer Menge in einem Bereich von 0,1 bis 10 Gew.-%, bezogen auf das Gesamtgewicht der Paste A, beinhaltet.

6. Kit nach einem der vorhergehenden Ansprüche, wobei die Schwermetallverbindung ausgewählt ist aus der Gruppe bestehend aus Kupfer(II)-hydroxid, basischem Kupfer(II)-carbonat und einer Mischung aus beiden.

7. Kit nach einem der vorhergehenden Ansprüche, wobei die Paste B die Schwermetallverbindung (b2) in einer Menge in einem Bereich von 0,0005 bis 0,5 Gew.-%, bezogen auf das Gesamtgewicht der Paste B, beinhaltet.

8. Kit nach einem der vorhergehenden Ansprüche, wobei der wenigstens eine in (a1) bzw. (b1) schwer- oder unlösliche Füllstoff (a3) bzw. (b3) ein partikuläres Polymer ist.

9. Kit nach einem der vorhergehenden Ansprüche, wobei der wenigstens eine in (a1) bzw. (b1) schwer- oder unlösliche Füllstoff (a3) bzw. (b3) ausgewählt ist aus der Gruppe bestehend aus vernetztem Poly(methylmethacrylat-co-methylacrylat), vernetztem Poly(methylmethacrylat) und einer Mischung dieser beiden Polymere.

10. Kit nach einem der vorhergehenden Ansprüche, wobei das wenigstens eine anorganische Halogenid-Salz (a4) bzw. (b4) Lithiumchlorid ist.

11. Kit nach einem der vorhergehenden Ansprüche, wobei Paste A, Paste B oder Paste A und Paste B das mindestens eine anorganische Halogenid-Salz (a4) bzw. (b4) in einer Menge in einem Bereich von 0,001 bis 7,5 Gew.-%, bezogen auf das Gesamtgewicht der Paste A bzw. B, beinhalten.

12. Kit nach einem der vorhergehenden Ansprüche, wobei Paste A, Paste B oder Paste A und Paste B ein in (a1) bzw. (b1) lösliches Polymer (a5) bzw. (b5) beinhalten.

13. Kit nach Anspruch 12, wobei das in (a1) bzw. (b1) lösliche Polymer (a5) bzw. (b5) ausgewählt ist aus der Gruppe bestehend aus Poly(methacrylsäure-methylester), Poly(methacrylsäureethylester), Poly-(methylmethacrylsäurepropylester), Poly(methacrylsäureisopropylester), Poly(methylmeth-acrylat-co-methylacrylat), Poly(styrol-co-methyl-meth-acrylat) und einer Mischung aus mindestens zwei dieser Polymere.

14. Kit nach einem der Ansprüche 1 bis 13, wobei Paste A 15 bis 85 Gew.-%, bezogen auf das Gesamtgewicht der Paste A, eines in (a1) schwer- oder unlöslichen Füllstoffs (a3) und Paste B weniger als 5 Gew.-%, bezogen auf das Gesamtgewicht der Paste B, eines in (b1) schwer- oder unlöslichen Füllstoffs (b3) beinhaltet.

15. Kit nach Anspruch 14, wobei Paste A ein in (a1) lösliches Polymer (a5) in einer Menge in einem Bereich von 1 bis 25 Gew.-%, bezogen auf das Gesamtgewicht der Paste A, und Paste B ein in (b1) lösliches Polymer (b5) in einer Menge in einem Bereich von 25 bis 85 Gew.-%, bezogen auf das Gesamtgewicht der Paste B, beinhaltet.

16. Kit nach einem der Ansprüche 1 bis 13, wobei Paste A 15 bis 85 Gew.-%, bezogen auf das Gesamtgewicht der Paste A, eines in (a1) schwer- oder unlöslichen Füllstoffs (a3) und Paste B 15 bis 85 Gew.-%, bezogen auf das Gesamtgewicht der Paste B, eines in (b1) schwer- oder unlöslichen Füllstoffs (b3) beinhaltet.

17. Kit nach Anspruch 16, wobei Paste A ein in (a1) lösliches Polymer (a5) in einer Menge in einem Bereich von 5 bis 50 Gew.-%, bezogen auf das Gesamtgewicht der Paste A, und/oder Paste B ein in (b1) lösliches Polymer (b5) in einer Menge in einem Bereich von 5 bis 50 Gew.-%, bezogen auf das Gesamtgewicht der Paste B, beinhaltet.

18. Verwendung eines Kits nach einem der vorhergehenden Ansprüche zur Herstellung einer Paste zur mechanischen Fixierung von Gelenkprothesen, zur Abdeckung von Schädeldefekten, zur Auffüllung von Knochenkavitäten, zur Femuroplastie, zur Vertebroplastie, zur Kyphoplastie, zur Herstellung von Spacern oder zur Herstellung von Trägermaterialien für die lokale Antibiotika-Therapie.

## Claims

1. Kit for the production of a bone cement comprising a paste A and a paste B, whereby
(a) paste A contains
(a1) at least one methacrylate monomer, and
(a2) at least one barbituric acid derivative as polymerisation initiator;
(b) paste B contains
(b1) at least one methacrylate monomer, and
(b2) at least one basic heavy metal compound, as polymerisation accelerator, selected from the group consisting of heavy metal salts and heavy metal complexes;
and whereby
i) paste B contains less than 0.01 % by weight of a peroxide, relative to the total weight of paste B,
ii) at least one of the pastes A and B contains, as component (a3) and/or (b3), at least one filling agent that is poorly soluble or insoluble in (a1) and/or (b1),
iii) at least one of the pastes A and B contains, as component (a4) and/or (b4), at least one inorganic halogenide salt, whereby
the at least one inorganic halogenide salt (a4) and/or (b4) is selected from the group consisting of an alkali halogenide, an alkaline earth halogenide, and a mixture of at least two of said inorganic halogenide salts, and
iv) whereby paste B contains less than 2% by weight of an organic acid that is soluble in monomer (b1), relative to the total weight of paste B.

2. Kit according to claim 1, whereby paste A and paste B contain the monomer (a1) and/or (b1) for radical polymerisation in an amount in the range of 15 to 85% by weight, each relative to the total weight of paste A and paste B, respectively.

3. Kit according to claim 1, whereby the barbituric acid derivative (a2) is selected from the group consisting of 1-monosubstituted barbiturates, 5-monosubstituted barbiturates, 1,5-disubstituted barbiturates, 1,3,5-trisubstituted barbiturates, and 1,3,5-tetrasubstituted barbiturates.

4. Kit according to any one of the preceding claims, whereby the at least one barbituric acid derivative (a2) is selected from the group consisting of 1-cyclohexyl-5-ethyl-barbituric acid, 1-phenyl-5-ethyl-barbituric acid, and 1,3,5-trimethyl-barbituric acid.

5. Kit according to any one of the preceding claims, whereby paste A contains the at least one barbituric acid derivative (a2) in an amount in the range of 0.1 to 10% by weight, relative to the total weight of paste A.

6. Kit according to any one of the preceding claims, whereby the heavy metal compound is selected from the group consisting of copper(II) hydroxide, basic copper(II) carbonate and a mixture of both.

7. Kit according to any one of the preceding claims, whereby paste B contains the heavy metal compound (b2) in an amount in the range of 0.0005 to 0.5% by weight, relative to the total weight of paste B.

8. Kit according to any one of the preceding claims, whereby the at least one filling agent (a3) and/or (b3) that is poorly soluble or insoluble in (a1) and/or (b1) is a particulate polymer.

9. Kit according to any one of the preceding claims, whereby the at least one filling agent (a3) and/or (b3) that is poorly soluble or insoluble in (a1) and/or (b1) is selected from the group consisting of cross-linked poly(methylmethacrylate-co-methylacrylate), cross-linked poly(methylmethacrylate) and a mixture of these two polymers.

10. Kit according to any one of the preceding claims whereby the at least one inorganic halogenide salt (a4) and/or (b4) is lithium chloride.

11. Kit according to any one of the preceding claims, whereby paste A, paste B or paste A and paste B contain the at least one inorganic halogenide salt (a4) and/or (b4) in an amount in the range of 0.001 to 7.5% by weight, relative to the total weight of paste A and/or B.

12. Kit according to any one of the preceding claims, whereby paste A, paste B or paste A and paste B contain a soluble polymer (a5) and/or (b5) that is soluble in (a1) and/or (b1).

13. Kit according to claim 12, whereby the soluble polymer (a5) and/or (b5) that is soluble in (a1) and/or (b1) is selected from the group consisting of poly(methacrylic acid methyl ester), poly(methacrylic acid ethyl ester), poly(methylmethacrylic _{[A1]}acid propyl ester, poly(methacrylic acid isopropyl ester), poly(methylmethacrylate-co-methylacrylate), poly(styrene-co-methylmethacrylate), and a mixture of at least two of these polymers.

14. Kit according to any one of the claims 1 to 13, whereby paste A contains 15 to 85% by weight, relative to the total weight of paste A, of a filling agent (a3) that is poorly soluble or insoluble in (a1) and paste B contains less than 5% by weight, relative to the total weight of paste B, of a filling agent (b3) that is poorly soluble or insoluble in (b1).

15. Kit according to claim 14, whereby paste A contains a polymer (a5) that is soluble in (a1) in an amount in the range of 1 to 25% by weight, relative to the total weight of paste A, and paste B contains a polymer (b5) that is soluble in (b1) in an amount in the range of 25 to 85% by weight, relative to the total weight of paste B.

16. Kit according to any one of the claims 1 to 13, whereby paste A contains 15 to 85% by weight, relative to the total weight of paste A, of a filling agent (a3) that is poorly soluble or insoluble in (a1) and paste B contains 15 to 85% by weight, relative to the total weight of paste B, of a filling agent (b3) that is poorly soluble or insoluble in (b1).

17. Kit according to claim 16, whereby paste A contains a polymer (a5) that is soluble in (a1) in an amount in the range of 5 to 50% by weight, relative to the total weight of paste A, and/or paste B contains a polymer (b5) that is soluble in (b1) in an amount in the range of 5 to 50% by weight, relative to the total weight of paste B.

18. Use of a kit according to any one of the preceding claims for the production of a paste for mechanical fixation of articular prostheses, for coverage of skull defects, for the filling of bone cavities, for femuroplasty, for vertebroplasty, for kyphoplasty, for the production of spacers or for the production of carrier materials for local antibiotics therapy.

## Revendications

1. Kit pour la fabrication d'un ciment osseux, présentant une pâte A et une pâte B, dans lequel
(a) la pâte A
(a1) contient au moins un monomère de méthacrylate et
(a2) au moins un dérivé d'acide barbiturique en tant qu'initiateur de polymérisation ;
(b) la pâte B
(b1) contient au moins un monomère de méthacrylate et
(b2) au moins un composé de métal lourd basique en tant qu'accélérateur de polymérisation qui est sélectionné parmi le groupe constitué de sels de métal lourd et de complexes de métal lourd ;
et dans lequel
i) la pâte B contient moins de 0,01 % en poids, par rapport au poids total de la pâte B, d'un peroxyde,
ii) au moins une des pâtes A et B contient en tant que composant (a3) ou (b3) au moins une charge difficilement soluble ou insoluble dans (a1) ou (b1),
iii) au moins une des pâtes A et B contient en tant que composant (a4) ou (b4) au moins un sel d'halogénure inorganique, dans lequel
l'au moins un sel d'halogénure inorganique (a4) ou (b4) est sélectionné parmi le groupe constitué d'un halogénure alcalin, d'un halogénure alcalino-terreux et d'un mélange d'au moins deux de ces sels d'halogénure inorganiques, et
iv) dans lequel la pâte B contient moins de 2 % en poids par rapport au poids total de la pâte B d'un acide organique qui est soluble dans le monomère (b1).

2. Kit selon la revendication 1, dans lequel la pâte A et la pâte B contiennent l'au moins un monomère polymérisable par voie radicalaire (a1) ou (b1) en une quantité dans une plage de 15 à 85 % en poids, à chaque fois par rapport au poids total de la pâte A ou de la pâte B.

3. Kit selon la revendication 1 ou 2, dans lequel le dérivé d'acide barbiturique (a2) est sélectionné parmi le groupe constitué de barbiturates 1-monosubstitués, de barbiturates 5-monosubstitués, de barbiturates 1,5-disubstitués, de barbiturates 1,3,5-trisubstitués et de barbiturates 1,3,5-tétrasubstitués.

4. Kit selon une des revendications précédentes, dans lequel l'au moins un dérivé d'acide barbiturique (a2) est sélectionné parmi le groupe constitué de l'acide 1-cyclohexyl-5-éthyl-barbiturique, l'acide 1-phényl-5-éthyl-barbiturique et l'acide 1,3,5-triméthyl-barbiturique.

5. Kit selon une des revendications précédentes, dans lequel la pâte A contient l'au moins un dérivé d'acide barbiturique (a2) en une quantité dans une plage de 0,1 à 10 % en poids, par rapport au poids total de la pâte A.

6. Kit selon une des revendications précédentes, dans lequel le composé de métal lourd est sélectionné parmi le groupe constitué de l'hydroxyde de cuivre(II), du carbonate de cuivre(II) basique et d'un mélange des deux.

7. Kit selon une des revendications précédentes, dans lequel la pâte B contient le composé de métal lourd (b2) en une quantité dans une plage de 0,0005 à 0,5 % en poids, par rapport au poids total de la pâte B.

8. Kit selon une des revendications précédentes, dans lequel l'au moins une charge (a3) ou (b3) difficilement soluble ou insoluble dans (a1) ou (b1) est un polymère particulaire.

9. Kit selon une des revendications précédentes, dans lequel l'au moins une charge (a3) ou (b3) difficilement soluble ou insoluble dans (a1) ou (b1) est sélectionnée parmi le groupe constitué du poly(méthylméthacrylate-co-méthylacrylate) réticulé, du poly(méthylméthacrylate) réticulé et d'un mélange de ces deux polymères.

10. Kit selon une des revendications précédentes, dans lequel l'au moins un sel d'halogénure inorganique (a4) ou (b4) est le chlorure de lithium.

11. Kit selon une des revendications précédentes, dans lequel la pâte A, la pâte B ou la pâte A et la pâte B contiennent l'au moins un sel d'halogénure inorganique (a4) ou (b4) en une quantité dans une plage de 0,001 à 7,5 % en poids, par rapport au poids total de la pâte A ou B.

12. Kit selon une des revendications précédentes, dans lequel la pâte A, la pâte B ou la pâte A et la pâte B contiennent un polymère (a5) ou (b5) soluble dans (a1) ou (b1).

13. Kit selon la revendication 12, dans lequel le polymère (a5) ou (b5) soluble dans (a1) ou (b1) est sélectionné parmi le groupe constitué de l'ester méthylique d'acide poly(méthacrylique), l'ester éthylique d'acide poly(méthacrylique), l'ester propylique d'acide poly-(méthylméthacrylique), l'ester isopropylique d'acide poly(méthacrylique), du poly(méthylméth-acrylate-co-méthylacrylate), du poly(styrène-co-méthyl-méth-acrylate) et d'un mélange d'au moins deux de ces polymères.

14. Kit selon une des revendications 1 à 13, dans lequel la pâte A contient 15 à 85 % en poids, par rapport au poids total de la pâte A, d'une charge (a3) difficilement soluble ou insoluble dans (a1) et la pâte B contient moins de 5 % en poids, par rapport au poids total de la pâte B, d'une charge (b3) difficilement soluble ou insoluble dans (b1).

15. Kit selon la revendication 14, dans lequel la pâte A contient un polymère (a5) soluble dans (a1) en une quantité dans une plage de 1 à 25 % en poids, par rapport au poids total de la pâte A, et la pâte B contient un polymère (b5) soluble dans (b1) en une quantité dans une plage de 25 à 85 % en poids, par rapport au poids total de la pâte B.

16. Kit selon une des revendications 1 à 13, dans lequel la pâte A contient 15 à 85 % en poids, par rapport au poids total de la pâte A, d'une charge (a3) difficilement soluble ou insoluble dans (a1) et la pâte B contient 15 à 85 % en poids, par rapport au poids total de la pâte B, d'une charge (b3) difficilement soluble ou insoluble dans (b1).

17. Kit selon la revendication 16, dans lequel la pâte A contient un polymère (a5) soluble dans (a1) en une quantité dans une plage de 5 à 50 % en poids, par rapport au poids total de la pâte A, et/ou la pâte B contient un polymère (b5) soluble dans (b1) en une quantité dans une plage de 5 à 50 % en poids, par rapport au poids total de la pâte B.

18. Utilisation d'un kit selon une des revendications précédentes pour la fabrication d'une pâte pour la fixation mécanique de prothèses articulées, pour le recouvrement de défauts crâniens, pour le remplissage de cavités osseuses, pour la fémoroplastie, pour la vertébroplastie, pour la kyphoplastie, pour la fabrication d'écarteurs ou pour la fabrication de matériaux de support pour l'antibiothérapie locale.
